(19) Europäisches Patentamt
European Patent Office
Office européen des brevets

(11) Publication number: **0 331 988 A1**

(12) **EUROPEAN PATENT APPLICATION**

(21) Application number: 89103237.7

(22) Date of filing: 24.02.89

(51) Int. Cl.[4]: C07C 149/46 , C07C 102/06 , C07K 1/08

(30) Priority: 08.03.88 JP 55856/88

(43) Date of publication of application: 13.09.89 Bulletin 89/37

(84) Designated Contracting States: CH DE FR GB IT LI NL

(71) Applicant: SANSHIN KAGAKU KOGYO CO., LTD.
150, Oaza Yanai
Yanai-shi Yamaguchi-ken(JP)

(72) Inventor: Kouge, Katsushige
777-3, Oaza Hiraoson Kirao-cho
Kumage-gun Yamaguchi-ken(JP)

(74) Representative: Denmark, James
c/o Bailey, Walsh & Co. 5 York Place
Leeds LS1 2SD Yorkshire(GB)

(54) **Sulfonium compounds, as well as process for producing active esters and amides used for the production of amides.**

(57) In the present invention an amide represented by the general formula:

$$W - \underset{\underset{O}{\|}}{C} - B - Y$$

is produced using a sulfonium salt represented by the general formula:

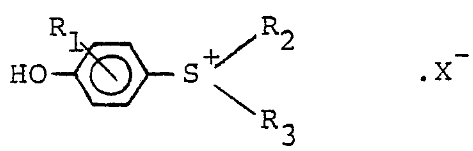

as the starting material, to synthesizing a novel compound, i.e., an active ester represented by the general formula:

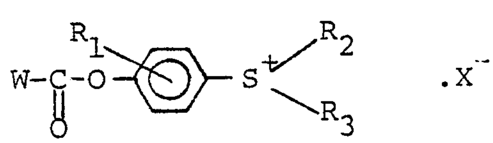

and further reacting the active ester with a nucleophilic agent represented by the general formula: H-B-Y wherein $R_1$ represents a hydrogen, an alkyl group or a halogen group; $R_2$ and $R_3$, which may be same or different, each represents an alkyl group; $X^-$ represents an anion typified by a halogen ion, a perchloric acid ion, a hydrogen sulfuric acid ion, a methylsulfuric acid ion, and a p-toluenesulfonic acid ion; W represents an alkyl group, alkoxy group

EP 0 331 988 A1

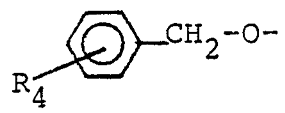 group,

in which $R_4$ represents a halogen, a hydrogen or a methoxy or a $C_1$ - $C_5$ alkyl group substituted with 1 - 3 halogen atoms, an aryl group or a fluorenylmethoxy group, or represents, together with $W-\underset{\underset{O}{\|}}{C}-$,

an amino acid, a peptide, or a protein group, in which the terminal amino group and the side chain group of the amino acid has or has not a protective group, B represents a substituted or not-substituted imino group, Y represents an organic group or B and Y join together to represent a heterocyclic amine. Alternatively H-B-Y represents an amino acid in which a proper protective group is or is not introduced into α-carboxyl group and/or side chain, or a peptide or protein in which a proper protective group is or is not introduced into the terminal carboxyl group and/or side chain.

# Sulfonium compounds, as well as process for producing active esters and amides used for the production of amides.

2. Field of the Invention and Description of Related Technologies

This invention relates to an active esterifying agent used for chemical synthesis of peptides, chemical modification of proteins and production of other amides useful in industries, as well as an active ester represented by a novel sulfonium salt, and a process for producing amides using such active ester.

For a chemical synthesizing process of physiologically active peptides such as favoring peptides typified by many hormone peptides and sweetenings, a chemical modification process of proteins which is the chemical means for immune response, elucidation of enzymatic functions or transformation of proteins, and also a chemical synthesizing process of other amides useful in industries, various methods have been studied for a long time. For example, a method using acid chlorides (E. Fisher, Chem. Ber., 36, 2094 - 2106 (1903)), a method using acid azides (J. Curtius Chem, Ber., 35, 3226 -3228(1902)), a method using dehydrating condensing agents such as dicyclohexylcarbodiimide (H.G. Khorana, J. Chem. Soc., 1952, 2081 - 2088), a mixed acid anhydride method (Th. Wieland, et al Justus Liebigs Annalen der Chemie, 569, 117 -121 (19560)), and an active ester method to promote nucleophilic substituting reaction by transforming carboxyl components to the active esters (M. Bodauszky, J. Amer. Chem. Soc., 81, 5688 - 5691 (1959); J. Kovacs, M.Q. Ceprini, Chemistry and Industry., 1965, 2100; G.W. Anderson, et al., J. Amer. Chem. Soc., 85, 3039 (1963); Japanese Patent Application OPI No. 166670/1985; Chemical Abstracts, 104 88559p : Chemical Abstracts s 103 1963935)) have been known.

For formation of a acid amide bond, particularly, tor the formation of peptide bond or for the preparation of the N-end protected amino acid in these various methods, the method using the acid chloride by E. Fisher described above has usually been employed. However, although the acid chloride method is excellent in the reactivity, it is inferior to other methods in view of the stability of the reagent. Particularly, the reagent undergoes hydrolysis during reaction in an aqueous solution. Accordingly, since the reaction requires excess amount of the reagent and because of the stimulating property of the reagent, it is difficult to handle. In this regard, the active ester method, differing from other methods, has a characteristic in which the reaction proceeds even when water is present in the reaction system, and is also an important reaction reagent in the field of chemical modification of proteins, as well as synthesis of peptides.

Namely, the active ester method means a process for producing an amide (RCONHR′) by reacting an organic compound (RCOOH) with an active esterifying assistant (X-OH) to form an active ester (RCOOX) and then causing an amide (R″$NH_2$) to act on this active ester, and has an advantage in which the reaction proceeds under relatively mild conditions.

As the active esters conventionally used for such purposes, esters with phenol derivatives such as p-nitrophenol and pentachlorophenol and esters with N-hydroxysuccinimide have been known. Namely, the reaction system of aminolysis, for example, by means of the active esters process is (I) + (II) → (III) + (IV) as follows.

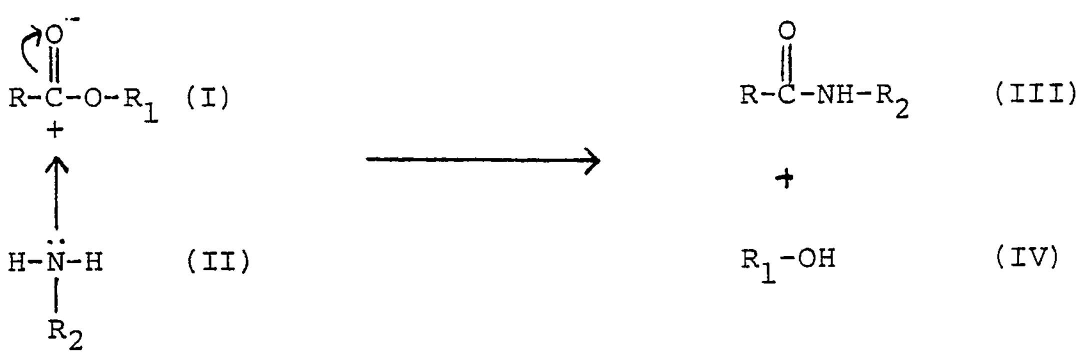
(I)
(II)
(III)
(IV)

In an active ester (I), generally, R activates the carbonyl carbon using a phenyl nucleus having electrophilic groups, for example, p-nitrophenyl or pentachlorophenyl. After completion of aminolysis, a hydroxyl compound (IV), the unreacted active ester (I) and an amine component (II) are naturally present as a mixture in the formed product (III). To take out only the desired product (III) from this state, each of (I), (II) and (IV) is required to be removed out of the reaction system. Generally, this mixture is dissolved in an

organic solvent having no compatibility with water such as ethyl acetate, (II) is first removed by separating and washing the solution using a diluted acidic aqueous solution, and then removing procedures of (IV) and (I) are started. With respect to removal of (IV), separation and washing using a minutely alkaline aqueous solution is generally conducted, but a complete removal cannot be achieved as the active ester (I) has a high hydrophobicity when $R_1$ is a phenyl nucleus or long chained alkyl group. Accordingly, to obtain a satisfactory purity, removal of impurity by recrystallization or chromatographic purification is required. Furthermore, since (I) is hardly dissolved in water and the trend is remarkable when R is a phenyl nucleus or long-chained alkyl, only recrystallization by an organic solvent is effective, for removal of (I). However, when the active ester (I) and the product (III) are similar in solubilities, it cannot be completely removed, which often causes reduction of purity.

Thus, as active esters more easily purified, hydroxyamide systems, for example, N-hydroxysuccinimide, are considered to be effective. However, although it has a merit in which, as N-hydroxysuccinimide is water-soluble in itself, it is easily removed by washing with water, the unreacted active esters (I) are not water-soluble in general, and thus, its removal must depend on recrystallization by organic solvents. Also, in this case, as the solubility is more similar to that of the product (III), compared with the case of phenol type active esters, a satisfactory result cannot be obtained for reaction yields in total. On the other hand, when the amine component which is an attacking reagent for forming carboxylic acid amide bond is a high molecular peptide and protein, the use of water is required, as the solvent for the amine component. However, in the reaction using each of the above-said reagents, as the active ester is hardly soluble to water, it must be dissolved and reacted in a mixed system of a solvent having a compatibility with water and, consequently, the amino component such as peptide or protein is inevitably deformed by the solvent. In the known literatures or in the present application, the active ester is referred to as "acylating agent", which is made in view of the amine of the abovementioned formula (II), since the reaction from (II) to (III) is the acylation. Further, the situation is the same in which a protective groups is introduced to the N-end such as in an amino acid. That is, the active ester is also effective in a reaction in which the active ester (I) as the protective group is brought into reaction with an amino acid (II) to form an amino acid (III) where the protective group is introduced at the N-position. As the example for the protective group in this case, there can be mentioned those in which R is tert-butyloxy group, benzyloxy group, 9-fluorenylmethoxy group, p-methoxybenzyloxy group etc.

3. Object and Summary of the Invention

An object of this invention is to provide a novel sulfonium compound, as well as to provide an active esterifying agent for rendering the active ester water-solubilized, and a water soluble active ester represented as a novel sulfonium compound.

Another object of this invention is to provide a producing process in which an amide, particularly, peptidic compound can be obtained at a high purity by using a water-solubilized active ester thereby progressing the active ester method moderately in water.

Among the sulfonium compounds of this invention represented by the general fomula:

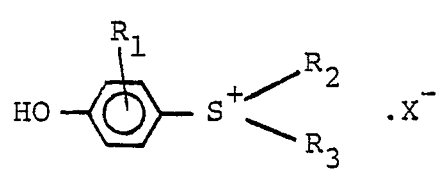

4-hydroxy-2-methylphenyldimethylsulfonium iodide is a publicly-known compound described in J. Amer. Chem. Soc., 80, 3425 (1958) and has been known as a water-soluble phenol derivative having a high acidity. However, there have been no examples of deriving this compound to the ester and also reporting its function as the active ester.

The sulfonium compound described above represented as the starting material for the active ester according to the present invention, that is, the active esterifying agent is formed as a reaction by-product after the active esterifying reaction. This is a water-soluble compound from its ionicity and properties of the substituting groups. Therefore, in separation and removal after reaction which has been regarded as a problem in the conventional active esters, it has also a characteristic in which it can be easily separated and removed from the desired product by washing with water. As the active esters of this invention themselves

are water-soluble in most cases, a system of water alone can be selected as the reaction solvent, even when amino acids, peptides and proteins which are water-soluble compounds are used as the nucleophilic agent. Further, the unreacted active ester itself can also be removed out of the system easily by washing with water. This is an important characteristic which is not possessed by the conventional active esters, and can become a means to solve the problem of deformation of peptides, etc. by organic solvents. Also, in synthesis of amides mainly composed of peptides in a system using general organic solvents, there is no difference in reaction yields between the active esters of this invention and the conventional active esters.

With respect to reactivity, the active esters of this invention afford reaction yields equal or more, compared with the conventional esters of p-nitrophenol, pentachlorophenol, N-hydroxysuccinimide, etc.

Furthermore, a significant characteristic of the active esters of this invention is that the active ester per se is water-soluble, and thus the reaction can be conducted in a solvent of water alone. This enables an easy acylating reaction of compounds in vivo such as amino acids, peptides, and proteins in aqueous solutions under moderate conditions which was conventionally impossible. Conventionally, when such reaction was conducted, functional groups and side chains not participating in the reaction were required to be protected by proper protective groups before conducting the reaction, but in the water-soluble active ester of this invention, as water can be used as the reaction solvent as described above, those groups of the main chains, side chains and functional groups having predetermined acid dissociation constant are protonated and substantially protected, and as a result, cnly α-amino groups of amino acids or peptides or terminal amino groups can be selectively acylated, while those groups of the main chains, side chains and functional groups having predetermined acid dissociation constant remain not protected. This is useful in the side chain acylation of lysine or ornithine. Further, in a case of introducing an amino acid having amino group on the side chain such as lysine or ornithine further into a peptide, etc., the amino group at the ε-position of lysine or δ-position of ornithine reacts in the same manner as the α-amino group. Accordingly, for synthesizing a peptide including lysine or ornithine, it is necessary that the ε-position of lysine or δ-position of ornithine is previously capped by a protective group represented by the acyl group so as not to be reacted. However, if usual acid halide is caused to react, both of the amino groups on the main and side chains are acylated to form a diacyl compound. In U.S. Patent No. 4126628, a water insoluble active ester is used for overcoming the foregoing drawback. However, since the active ester is water insoluble, the reactivity is low. As the conventional method of capping the ε-position of lysine or δ-position of ornithine by the acyl group so as not to cause them into reaction, the synthesis has been made by way of a copper salt as described as a comparative example in reference example 3. However, both of the copper salt and hydrogen sulfide undergo industrial limits. When the reaction is conducted by using the acylating agent according to the present invention at a pH of 5 or above and with 1-2 moles of sulfonium compound per mole of lysine or ornithine only the side chain acylated compound can be obtained selectively in one step reaction. Furthermore, for the chemical modification of proteins, since the reaction can be conducted without using an organic solvent in contrast to the case of the conventional active ester, no protein modification occurs. This is one of the features which cannot be found in the conventional active ester.

The active esters of this invention are not limited only by using water as the reaction solvent, and even if other general solvents having a compatibility with water or solvents having no compatibility with water are employed, they exhibit the effect sufficiently. For example, in a two layer heterogeneous system of chloroform, dichloromethane or ethyl acetate which can dissolve the sulfonium salt of this invention and water, the reaction also proceeds smoothly.

Also, it can be applied to not only formation of peptide bonds but also synthesis of amides useful in other industries such as medicine, agricultural agents, etc., introduction of protection groups to amino(imino) groups in amino acids, peptides, etc.

4. Detailed Description of Preferred Embodiment of the Invention

The novel sulfonium compound and the active esterifying agent for synthesizing active ester represented as the novel sulfonium compound of this invention comprises a sulfonium salt represented by the general formula:

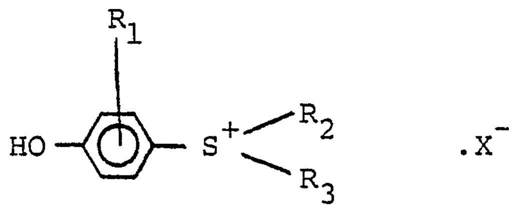 (a)

where $R_1$ represents a hydrogen, an alkyl group, or a halogen group; $R_2$ and $R_3$, which may be same or different, each represents an alkyl group; and $X^-$ represents an anion typified by a halogen ion, a perchloric acid ion, a hydrogensulfuric acid ion, a methylsulfuric acid ion, and a p-toluenesulfonic acid ion.

The preferred concrete examples of the active esterifying agent include 4-hydroxyphenyldimethylsulfonium perchlorate, 4-hydroxyphenyldimethylsulfonium p-toluenesulfonate, 4-hydroxyphenyldimethylsulfonium methylsufate, 4-hydroxyphenyldimethylsulfonium hydrogensulfate and the like.

The active ester derived from these active esterifying agents comprises a novel sulfonium compound represented by the general formula:

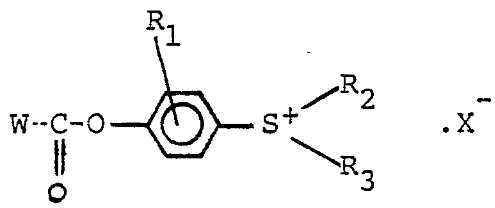 (b)

wherein $R_1$ - $R_3$ and $X^-$ are the same defined in the above general formula (a): and W represents an alkyl group, alkoxy group,

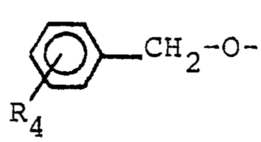 **group,**

(in which $R_4$ represents a halogen, a hydrogen or a methoxy), or a $C_1$ - $C_5$ alkyl group substituted with 1 - 3 halogen atoms, an aryl group or a fluorenylmethoxy group, or represents, together with $W-\underset{\underset{O}{\|}}{C}-$, an amino acid, a peptide, or a protein group, in which the terminal amino group and the side chain group of the amino acid has or has not a protective group.

Such an active ester can be synthesized easily also by the reaction between 4-hydroxyphenyl dialkyl sulfoniums as the active esterifying agent represented by the general formula (a) described above and an acid chloride or an acid anhydride represented by W-CO-C1 or W-CO-O-CO-W (in which W represents an alkyl group, an alkoxy group, a $C_1$ - $C_5$ alkyl group substituted with 1 - 3 halogen atoms, an aryl group, a fluorenylmethoxy group,

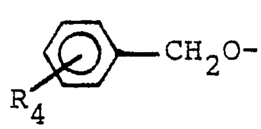

group in which $R_4$ represents a hydrogen, a halogen or a methoxy group), or reaction between the active esterifying group represented by the general formula (a) described above and W-COOH in which W has the same meanings as described above and dicyclohexylcarbodimide. The active ester in which W is the residue of an amino acid together with W-CO-in the general formula (b) described above can be synthesized easily by synthesizing 4-hydroxyphenyl dialkyl sulfoniums of the active esterifying agent represented by the general formula (a) described above and an amino acid derivative represented by

$$W'\text{-NH-}\overset{R}{\underset{}{C}}\text{H-COOH} \quad (c)$$

(wherein W′ represents a hydrogen atom or a protective group of α-amino group of an amino acid, R represents an amino acid side chain having or not having a protective group, and $R_1$-$R_3$ and $X^-$ are the same as defined in the above general formula (a)), by means of the mixed acid anhydride method, or by condensing them under action of dicyclohexylcarbodiimide. The situation is similar where W represents a peptide or a protein having two or more amino acids.

When a proper nucleophilic agent represented by the general formula H-B-Y is acted (reacted) on the active ester derived from the thus synthesized phenol sulfonium salt (ester sulfonium salt; represented by the above general formula (b)) as the substrate, peptide derivatives, chemical modified products of proteins, or other industrially useful amides represented by the general formula:

$$W\text{-}\underset{\underset{O}{\|}}{C}\text{-B-Y} \quad (d)$$

can be easily obtained in high yield and high purity. The reaction can also be progressed in a case where W is a hydrophobic group such as a long-chained alkyl group.

In the general formula H-B-Y described above, B represents a substituted or non-substituted imino group, Y represents an organic group, or B and Y join together to represent a heterocyclic amine typically represented by morpholine, piperidine or proline. Alternatively, H-B-Y represents an amino acid in which a proper protective group is or is not introduced into the α-carboxyl group and/or side chain, or a peptide or a protein in which a proper protective group is introduced or not introduced into the terminal carboxyl group and/or side chain.

The reaction of the active ester (ester sulfonium salt) represented by the above general formula (b) with the nucleophilic agent represented by the above general formula H-B-Y is conducted under conditions such as in water alone, in an organic solvent having a compatibility with water alone, in a mixed systems of the above two, in an organic solvent having no compatibility with water alone, or in two phase system of an organic solvent having no compatibility with water and water.

This invention is further illustrated in more detail by examples, but the compounds of this invention and the utility thereof are not limited only by the described examples.

## Synthetic Example 1

### Synthesis of 4-(benzyloxycarbonyloxy)phenyl dimethylsulfonium methylsulfate

In 20 ml of acetonitrile was dissolved 2.6 g of 4-hydroxyphenyldimethylsulfonium methylsulfate, and 1.6 ml of benzyloxycarbonyl chloride and 1.4 ml of triethylamine were dropwise added thereto under stirring at room temperature. The reaction mixture was filtered to remove the deposited triethylamine hydrochloride. The filtrate was concentrated under reduced pressure and the residue was crystallized by adding ether.

| | | | |
|---|---|---|---|
| Yield | 3.8 g (95 %) | | |
| m.p. | 76 - 80 °C | | |
| IR | 1760 $cm^{-1}$ (C=0) | | |
| Elem. anal. value | C: 51.33 %; | H: 5.11 %; | S: 15.91 % |
| (Theoretical value) | (51.01) | (4.99) | (15.99) |

## Synthetic Example 2

### Synthesis of 4-(benzyloxycarbonyloxy)-2-methylphenyl dimethylsulfonium perchlorate

In 30 ml of acetonitrile was dissolved 2.68 g of 4-hydroxy-2-methylphenyldimethylsulfonium perchlorate, and 1.6 ml of benzyloxycarbonyl chloride and 1.4 ml of triethylamine were dropwise added thereto under stirring at room temperature. The reaction mixture was filtered to remove the deposited triethylamine hydrochloride. The filtrate was concentrated under reduced pressure and the residue was crystallized by

adding ether.

| | | | |
|---|---|---|---|
| Yield | 3.5 g (91 %) | | |
| m.p. | 129 - 133 °C | | |
| IR | 1760 $cm^{-1}$ (C=0) | | |
| Elem. anal. value | C: 49.37 %; | H: 4.15 %; | S: 8.31 % |
| (Theoretical value) | (49.45) | (4.37) | (8.24) |

Synthetic Example 3

Synthesis of 4-acetoxyphenyldimethylsulfonium methylsulfate

In 30 ml of acetonitrile was dissolved 2.6 g of 4-hydroxyphenyldimethylsulfonium methylsulfate, and 0.8 ml of acetyl chloride and 1.4 ml of triethylamine were dropwise added thereto. The reaction mixture was filtered to remove the deposited triethylamine hydrochloride. The filtrate was concentrated under reduced pressure and the residue was crystallized by adding ether.

| | | | |
|---|---|---|---|
| Yield | 2.8 g (92 %) | | |
| m.p. | 84 - 86 °C | | |
| IR | 1760 $cm^{-1}$ (C=0) | | |
| Elem. anal. value | C: 42.54 %; | H: 4.99 %; | S: 21.05 % |
| (Theoretical value) | (42.87) | (5.19) | (20.81) |

Synthetic Example 4

Synthesis of 4-(tert-butyloxycarbonylphenylalanyloxy) phenyldimethylsulfonium methylsulfate

In 80 ml of acetonitrile were dissolved 2.65 g of tert-butyloxyphenylalanine and 2.66 g of 4-hydroxyphenyldimethylsulfonium methylsulfate, and dicyclohexyl carbodiimide was added thereto with stirring under ice-cooling. The reaction mixture was further stirred for 2 hours under ice-cooling and for 1 hour at room temperature, and then the dicyclohexyl urea was filtered off. The filtrate was concentrated under reduced pressure and the residue was crystallized by adding ether. This crystalline product (Hygroscopicity) gave 1 spot by TLC.

| | |
|---|---|
| Yield | 4.3 g(84 %) |
| IR | 1680 $cm^{-1}$ (C=0) |
| Rf | 0.51 (Butanol:Water:Pyridine:Acetic acid 4:1:1:2) |

Synthetic Example 5

Synthesis of 4-(benzyloxycarbonylalanyloxy)phenyl dimethylsulfonium methylsulfate

In 20 ml of acetonitrile was dissolved 2.24 g of benzyloxycarbonylalanine. After cooling the reaction mixture to -5 °C, 1.4 ml of triethylamine was added, and further 1 ml of ethyl chloroformate was dropwise added thereto. After 10 minutes, 50 ml of acetonitrile solution of 2.66 g of 4-hydroxyphenyldimethylsulfonium methylsulfate was gradually added thereto. The reaction mixture was stirred at 0 °C for 2 hours and

then at room temperature for 8 hours. The resulting reaction mixture was filtered, the filtrate was concentrated under reduced pressure, and the residue was crystallized by adding ether. This crystalline product (Hygroscopicity) afforded 1 spot by TLC.

| | |
|---|---|
| Yield | 3.7 g (78 %) |
| IR | 1680 $cm^{-1}$ (C=0) |
| Rf | 0.50 (Butanol:Water:Pyridine:Acetic acid 4:1:1:2) |

Synthetic Examples 6 - 12

Synthesis of 4-(palmitoyloxy)phenyldimethylsulfonium methylsulfate

In 20 ml of acetonitrile was dissolved 2.6 g of 4-hydroxyphenyldimethylsulfonium methylsulfate, and 3.3 ml of palmitic acid chloride and 1.4 ml of triethylamine were dropwise added thereto under stirring at a room temperature. The reaction mixture was filtered to remove the deposited triethylamine hydrochloride. The filtrate was concentrated under a reduced pressure and the residue was crystallized by adding ether.

The result of the synthesis and the result of the synthesis for various sulfonium compounds synthesized by the same method are shown in the table below.

Result for the synthesis of sulfonium compound

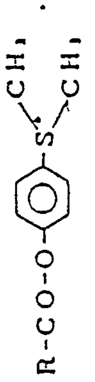 · $CH_3SO_4^-$

| Synthetic Example | R | Yield (%) | Melting point (°C) | Elementrary analysis Experimental value (Theoretical value) C% | H value |
|---|---|---|---|---|---|
| " 7 | $CH_3(CH_2)_3-$ | 81 | 88~90 | 47.59 (47.98 | 6.30 6.28) |
| " 8 | $CH_3(CH_2)_4-$ | 80 | 82~84 | 49.32 (49.46 | 6.47 6.59) |
| " 9 | $CH_3(CH_2)_8-$ | 93 | 84~85 | 54.11 (54.30 | 7.58 7.61) |
| " 10 | $CH_3(CH_2)_{10}-$ | 74 | 82~84 | 56.43 (56.22 | 7.99 8.02) |
| " 11 | $CH_3(CH_2)_{12}-$ | 81 | 80~83 | 57.84 (57.95 | 8.12 8.39) |
| " 6 | $CH_3(CH_2)_{14}-$ | 76 | 76~78 | 59.12 (59.49 | 8.43 8.71) |
| " 12 | $CH_3(CH_2)_{16}-$ | 88 | 85~87 | 60.71 (60.86 | 8.98 9.00) |

Synthetic Example 13

Synthesis of 4-(9-fluorenylmethoxycarbonyloxy)phenyl dimethylsulfonium methylsulfate

In 200 ml of acetonitrile was dissolved 13.3 g (0.05 mol) of 4-hydroxyphenyldimethylsulfonium methylsulfate, and 7 ml (0.05 mol) of triethylamine were dropwise added thereto under ice-cooling and stirring. After stirring for 20 min at that temperature, 15.5 g (0.06 mol) of 9-fluorenylmethoxycarbonyl chloride was added gradually and further stirred for 3 hours. The reaction mixture was filtered and the filtrate was concentrated under a reduced pressure and the residue was crystallized by adding ethyl acetate.

| | | |
|---|---|---|
| Yield<br>m.p.<br>IR | 20.7 g (85.0 %)<br>76 - 80 °C<br>1740 $cm^{-1}$, 1780 $cm^{-1}$ (C=0) | |
| Elementary analysis | | |
| Theoretical value $C_{24}H_{24}O_7S_2$<br>Measured value | C: 59.03 %;<br>C: 59.25 %; | H: 4.92%<br>H: 4.90 % |

Synthetic Example 14

Synthesis of 4-(p-methoxybenzyloxycarbonyloxy)phenyldimethylsulfonium methylsulfate

In 20 ml of acetonitrile was dissolved 2.6 g of 4-hydroxyphenyldimethylsulfonium methylsulfate, and 2.0 g of p-methoxybenzyloxycarbonyl chloride and 1.4 ml of triethylamine were dropwise added thereto under stirring at room temperature. The reaction mixture was filtered to remove the deposited triethylamine hydrochloride. The filtrate was concentrated under reduced pressure and the residue was crystallized by adding ether.

| | | |
|---|---|---|
| Yield | 2.9 g (70 %) | |
| m.p. | 106 - 108 °C | |
| IR | 1760 $cm^{-1}$ (C=0) | |
| Elemental anal. value | C: 49.73 % | H: 5.24 % |
| (Theoretical value) | C: 50.22 % | H: 5.15 % |

Working Examle 1 (Acylating reaction in aqueous solution)

Synthesis of benzyloxycarbonylglycine

In 20 ml of water was dissolved 4.0 g of 4-(benzyloxycarbonyloxy)phenyldimethylsulfonium methylsulfate prepared in Synthetic Example 1, and 20 ml of an aqueous solution of 0.75 g of glycine and 1.4 ml of triethylamine was dropwise added thereto under stirring at room temperature. The reaction mixture was further stirred for 8 hours at room temperature and adjusted to pH 2 by adding 2 % HC1. The aqueous solution was extracted twice with 50 ml of ethyl acetate. The ethyl acetate layer was dried and concentrated under reduced pressure, and ether was added to the obtained residue to afford a white crystalline product.

| | |
|---|---|
| Yield | 1.78 g (85.0 %) |
| m.p. | 119 - 120 °C (Literature value 120 °C) |

Working Example 2 (Acylating reaction in aqueous solution)

Synthesis of N-acetylphenylalanine

In 30 ml of water was dissolved 3.1 g of 4-acetoxy phenyldimethysulfonium methysulfate prepared in Synthetic Example 3, and 20 ml of an aqueous solution of 1.64 g of phenylalanine and 1.4 ml of triethylamine was dropwise added thereto under stirring at room temperature. The reaction mixture was further stirred for 12 hours at room temperature and adjusted to pH 2 by adding 2 % HC1. The aqueous

solution was extracted twice with 50 ml of ethyl acetate. The ethyl acetate layer was washed with water, dried, and concentrated under reduced pressure, and ether was added to the obtained residue to afford a white crystalline product.

| | |
|---|---|
| Yield | 1.4 g (70 %) |
| m.p. | 167 - 168° C (Literature value 168° C) |
| [α] D | +42° (Literature value +47°) |

Working Example 3 (Acylating reaction in aqueous solution)

Synthesis of benzyloxycarbonylarginine

In 30 ml of water was dissolved 4.0 g of 4-(benzyloxycarbonyloxy)phenyldimethylsulfonium methylsulfate prepared in Synthetic Example 1, and 20 ml of an aqueous solution of 1.74 g of arginine was dropwise added thereto under stirring at room temperature. The reaction mixture was further stirred for 10 hours at room temperature and then cooled to 5° C to deposit white crystals. The crystalline product was filtered and washed with water to obtain the desired product.

| | |
|---|---|
| Yield | 2.3 g (75 %) |
| m.p. | 174 - 175° C (Literature value 175° C) |

Working Example 4 (Acylating reaction in aqueous solution)

Synthesis of benzyloxycarbonylalanyglycine

In 20 ml of water was dissolved 4.33 g of 4-(benzyloxycarbonylalanyloxy)phenyldimethylsulfonium methylsulfate prepared in Synthetic Example 5, and 20 ml of an aqueous solution of 0.68 g of glycine and 1.28 ml of triethylamine was dropwise added thereto under stirring at room temperature. The reaction mixture was stirred for 12 hours at room temperature and adjusted to pH 2 by adding 2 % HC1. The aqueous solution was extracted twice with 100 ml of ethyl acetate. The ethyl acetate layer was dried and concentrated under reduced pressure, and ether was added to the obtained residue to afford white crystals.

| | |
|---|---|
| Yield | 1.58 g (61.9 %) |
| m.p. | 134.1 - 135.3° C |
| [α] D | -17.5° (cl. Alc) |

Working Example 5 (Synthesis of peptide in aqueous solution)

Synthesis of benzyloxycarbonylphenylalanylproline

In 30 ml of water was dissolved 5.47 g of 4-(benzyloxycarbonylphenylalanyloxy)-phenyldimethylsulfonium methylsulfate obtained by a synthesizing method according to Synthetic Example 5, and 20 ml of an aqueous solution of 1.15 g of proline and 1.4 ml of triethylamine was dropwise added thereto under stirring. The reaction mixture was further stirred for 12 hours at room temperature, adjusted to pH 2 by adding 2 % HC1, and extracted twice with 50 ml of ethyl acetate. The ethyl acetate layer was washed with water, dried, and concentrated under reduced pressure, and the obtained residue was

recrystallized by adding ether. This crystalline product indicated only the peak of the desired product by HPLC.

| | |
|---|---|
| Yield | 3.25 g (82 %) |
| m.p. | 105 - 106° C (Literature value 106° C) |
| [α] D | -63° (Literature value -64°) |

Working Example 6 (Synthesis of peptide in aqueous solution)

Synthesis of benzyloxycarbonylalanylvaline

In 30 ml of water was dissolved 4.72 g of 4-(benzyloxycarbonylalanyloxy)phenyldimethylsulfonium methylsulfate obtained by the method of Synthetic Example 5, and 20 ml of an aqueous solution of 1.17 g of valine and 1.4 ml of triethylamine was dropwise added thereto under stirring. The reaction mixture was stirred overnight at room temperature, adjusted to pH 2 by adding 2 % HC1, and extracted twice with 50 ml ethyl acetate. The ethyl acetate layer was washed with water, dried and concentrated under reduced pressure, and the residue was recrystallized by adding ether. This crystalline product indicated only the peak of the desired product by HPLC.

| | |
|---|---|
| Yield | 2.6 g (81 %) |
| m.p | 123 - 124° C (Literature value 121 - 124° C) |
| [α] D | -12° (Literature value -12°) |

Working Example 7 (Synthesis of peptide in aqueous solution)

Synthesis of tert-butyloxycarbonylmethionylarginylphenylalanylamide

In 20 ml of water was dissolved 2.01 g of arginylphenylalanylamide hydrochloride, and this aqueous solution was adjusted to pH 7.4 by adding triethylamine. To the reaction mixture was dropwise added 5 ml of an aqueous solution of 2.5 g of 4-(tert-butyloxycarbonylmethionyloxy)phenyldimethylsulfonium methylsulfate under stirring at room temperature, and the reaction mixture was maintained at pH 7.4 for 2 hours. As an oil was deposited from the reaction mixture, this oil was extracted with 50 ml of ethyl acetate. The ethyl acetate layer was washed with water, dried and then concentrated under reduced pressure, and the obtained residue was crystallized by adding ether. This crystalline product gave 1 spot by TLC and also indicated a positive Sakaguchi reaction. When this was hydrolyzed using 6N HC1 and analyzed by means of an amino acid analyzer, Met:Arg:Phe was 1:1.01:0.99, respectively.

| | |
|---|---|
| Yield | 3.47 g (65 %) |
| m.p | 84 - 85° C |
| [α] D | -21° (Literature value -21°) |

Working Example 8 (Reaction in two layer system of chloroform-water)

Synthesis of benzyloxycarbonylalanylvaline

In 50 ml of chloroform was dissolved 4.14 g of 4-(benzyloxycarbonylalanyloxy)phenyldimethylsulfonium

p-toluenesulfonate obtained by the method of Synthetic Example 5, and 20 ml of an aqueous solution of 0.91 g of valine and 1.1 ml of triethylamine was dropwise added thereto under stirring at room temperature. The reaction mixture was stirred for 8 hours at room temperature and then separated. The chloroform layer was washed with water, dried, and concentrated under reduced pressure, and ether was added to the residue to afford white crystals.

| | |
|---|---|
| Yield | 2.42 g (57.0 %) |
| m.p | 121.5 - 124.2° C |
| [α] D | -12° (cl. Alc) |

Working Example 9

Synthesis of ε-benzyloxycarbonyllysine

A pH electrode was put in a solution in which 14.6 g of lysine was dissolved in 100 ml water, and 60 g (1.5 mol equivalent against lysine) of 4-benzyloxycarbonyloxyphenyldimethylsulfonium methylsulfate synthesized in Synthetic Example 1 was added thereto under stirring at 20° C. As the pH became lowered as the reaction proceeded, 1M sodium carbonate solution was dropwise added thereto so as to make pH 8.0. While pH was maintained at 8.0 for 4 hours, the deposited white crystals were filtered, washed with water and methanol successively, and dried to afford 19.6 g (70 % of the theoretical value) of the desired product.

| | |
|---|---|
| m.p. | 255° C (Literature value m.p 253 - 255° C) |
| [α] D | +14° (cl. 1N-HC1; Literature value +14.4°) |

Working example 10

Synthesis of δ-benzyloxycarbonylornithine

A pH electrode was put in a solution in which 16.9 g of ornithine hydrochloride was dissolved in 100 ml of water, and 60 g (1.5 mol equivalent against ornithine) of 4-(benzyloxycarbonyloxy)-phenyldimethylsulfonium methylsulfate synthesized in Synthetic Example 1 was added thereto under stirring at 20° C. The pH of the reaction system was maintained 8.0 by 1N-NaOH and the reaction mixture was reacted for 4 hours. The deposited crystals were filtered, washed with water and methanol successively, and dried to afford 20.7 g (78 % of the theoretical value) of the desired product.

| | |
|---|---|
| m.p | 250-3° C (Literature value 253-5° C) |
| [α] D | +12° (cl. 1N-HC1: Literature value +13.1°) |

The presence of bis(benzyloxycarbonyl)ornithine was not recognized in the obtained crystalline product by means of thin layer chromatography.

Working Example 11

Synthesis of ε-tert-butyloxycarbonyllysine

A pH electrode was put in a solution of 14.6 g of lysine dissolved in 100 ml of water, and 55 g (1.5 mol equivalent against lysine) of 4-tert-butyloxycarbonyloxyphenyldimethylsulfonium methylsulfate was added

thereto under stirring at 20° C. The reaction was carried out at pH 8.0 according to the method of Working Example 10 to afford 17.7 g (72 % of the theoretical value) of the desired product.

| | |
|---|---|
| m.p | 250 - 255° C (Literature value 237 - 255° C) |
| [α] D | +6° (cl. 2N-$NH_4OH$; Literature value +4.7°) |

The presence of bis(tert-butyloxycarbonyl)lysine was not recognised in the obtained crystalline product by means of thin layer chromatography.

Working Example 12

Synthesis of N-ε-palmitoyllysine

Into 50 ml of water was dissolved 1.83 g of lysine hydrochloride and stirred with addition of 5 ml of 2N-NaOH at a room temperature. A pH electrode was put to a reactor and 5 g of 4-(palmitoyloxy)-phenyldimethylsulfonium methylsulfate synthesized in Synthetic Example 6 was added gradually at pH 11.3. During reaction, the pH was maintained at 11.3 with 2N-NaOH for two hours. Deposited white crystals were filtered and washed with water and ethanol.

The results are shown in the following table. Further, the result for synthesizing N-ε-acyllysine in the same reaction as in the abovementioned example using other acylating agents (varying R in the general formula) are also shown collectively in the following table.

Result for the synthesis of N-ε-acyllysine

| Working Example No. | Acylating agent R | Yield (%) | Melting point (℃) | $[\alpha]_D^{20}$ (1N-KOH) | Elementary analysis Experimental (Theoretical value %) C value | H | N |
|---|---|---|---|---|---|---|---|
| 13 | $CH_3(CH_2)_3-$ | 80 | 240 | +4.0 | 57.19 (57.41) | 9.55 (9.56) | 12.01 (12.17) |
| 14 | $CH_3(CH_2)_4-$ | 84 | 245 | +3.8 | 57.67 (57.91) | 10.39 (10.41) | 9.37 (9.64) |
| 15 | $CH_3(CH_2)_8-$ | 73 | 232~235 | +4.2 | 63.48 (63.96) | 10.72 (10.73) | 9.21 (9.32) |
| 16 | $CH_3(CH_2)_{10}-$ | 85 | 230~233 | +3.5 | 65.47 (65.81) | 11.01 (11.05) | 8.19 (8.52) |
| 17 | $CH_3(CH_2)_{12}-$ | 81 | 220~222 | +3.3 | 67.11 (67.22) | 11.28 (11.31) | 7.59 (7.85) |
| 12 | $CH_3(CH_2)_{14}-$ | 88 | 231~233 | +3.1 | 68.59 (68.70) | 11.44 (11.53) | 7.21 (7.28) |
| 18 | $CH_3(CH_2)_{16}-$ | 77 | 240 | +1.5 | 69.61 (69.85) | 11.70 (11.72) | 6.80 (6.79) |

Working Example 19 Acylating reaction in aqueous solution

Synthesis of N-palmitoylglycine

Into 20 ml of water, was dissolved 5 g of 4-(palmitoyloxy)phenyl dimethylsulfonium methyl sulfate and 20 ml of an aqueous solution comprising 0.75 g of glycine and 1.4 ml of triethylamine was added dropwise under stirring at a room temperature. They were further stirred at the room temperature for 8 hours and 2% HC1 was added to the reaction solution to adjust pH to 2. The aqueous solution was extracted twice each time with 50 ml of ethyl acetate. Then, the ethyl acetate layer was dried and ether was added to the residue obtained by concentration under a reduced pressure to obtain white crystals.

| | | | |
|---|---|---|---|
| Yield<br>m.p | 2.42 g (85.0 %)<br>107 - 109 °C | | |
| Elementary analysis | | | |
| Theoretical value $C_{18}H_{35}NO_3$<br>Measured value | C: 75.88 %;<br>C: 75.37 %; | H: 1.51 %;<br>H: 1.27 %; | N: 4.91 %<br>N: 4.95 % |

Working Example 20

Synthesis of N-9-fluorenylmethoxycarbonyl amino acid

Into 21 ml of an aqueous 10 % solution of sodium carbonate, various kinds of amino acids (0.01 mol) were dissolved and, while stirring at a room temperature, 4.88 g (0.01 mol) of 4-(9-fluorenylmethoxycarbonyloxy)phenyldimethylsulfonium methylsulfate as synthesized in Synthetic Example 13 was added and reacted at a room temperature for 12 hours. The reaction solution was rendered acidic with addition of 6N-HC1 and then extracted with ethyl acetate. The ethyl acetate layer was dried with anhydrous sodium sulfate and petroleum ether was added to the residue obtained by distillation under a reduced pressure to crystallize. The results are shown in the following table.

Synthesis of 9-fluorenylmethoxycarbonyl amino acid by water soluble 9-fluorenylmethoxtcarbonylating agent

| Starting amino acid | Yield (%) | Property of product | | Elementary analysis Experimental value (Theoretical value) % | | |
|---|---|---|---|---|---|---|
| | | Melting point (°C) | $[\alpha]_D^{20}$ (Solvent) | C | H | N |
| L-alanine | 80.0 | 143~144 | −4.0 (ethyl acetate) | 69.45 (69.33) | 5.47 (5.41) | 4.50 (4.61) |
| L-phenyl alanine | 84.0 | 184~186 | +12.0 (ethyl acetate) | 74.52 (74.33) | 5.43 (5.21) | 3.62 (3.58) |
| Glycine | 88.0 | 176~177 | — | 68.71 (68.68) | 5.65 (5.51) | 4.71 (4.80) |
| L-valine | 85.0 | 143~145 | −17.5 (DMF) | 70.82 (70.78) | 6.19 (6.03) | 4.13 (4.21) |

Working Example 21

Synthesis of p-methoxybenzyloxycarbonylglycine

In 20 ml of water was dissolved 4.3 g of 4-(p-methoxybenzyloxycarbonyloxy)phenyldimethylsulfonium methylsulfate prepared in Synthetic Example 14, and 20 ml of an aqueous solution of 0.75 g of glycine and 1.4 ml of triethylamine was dropwise added thereto under stirring at room temperature. The reaction mixture was stirred for 12 hours at room temperature and adjusted to pH 2 by adding 2% HC1. The aqueous solution was extracted twice with 100 ml of ethyl acetate. The ethyl acetate layer was dried and concentrated under reduced pressure, and ether was added to the obtained residue to afford white crystals.

| | |
|---|---|
| Yield | 2.25 g (94 %) |
| m.p | 94 - 96° C |

Reference Example 1 (Reaction in two layer system of chloroform-water using p-nitrophenyl ester)

Synthesis of benzyloxycarbonylglycine

In 50 ml of chloroform was dissolved 2.73 g of p-nitrophenylbenzyloxy carbonate, and 20 ml of an aqueous solution of 0.75 g of glycine and 1.4 ml of triethylamine was dropwise added thereto under stirring at room temperature. The reaction mixture was stirred for 8 hours at room temperature and then separated. The chloroform layer was washed with 1N aqueous ammonia and water, dried and concentrated under reduced pressure, and ether was added to the obtained residue to crystallize the desired product. The yield was 0.12 g and only 10 % of the theoretical amount was obtained.

Reference Example 2 (Suspending reaction in water using ester of N-hydroxysuccinimide)

Synthesis of benzyloxycarbonylglycine

In 50 ml of water was suspended 2.5 g of N-(benzyloxycarbonyloxy)succinimide, and the suspension was stirred for 1 hour at room temperature. To this suspension was added 20 ml of an aqueous solution of 0.75 g of glycine and 1.4 ml of triethylamine, and the reaction mixture was stirred for 12 hours as it was, but the desired product, benzyloxycarbonylglycine, could not be obtained.

Reference Example 3 (According to the conventional technology)

Synthesis of ε-benzyloxycarbonyllysine

In 320 ml of hot water was dissolved 11 g of lysine hydrochloride. To this was added 24 g of basic copper carbonate, and the reaction mixture was boiled for 10 minutes and filtered as it remained hot. The filtrate was allowed to cool, and 10 g of sodium hydrogencarbonate and 12 ml of benzyloxycarbonylchloride were divided into four, each of which was added thereto every 10 minutes. After stirring for 3 hours, the precipitate was filtered and washed with water and ethanol successively. Then the precipitate was dispersed in 250 ml of water and dissolved by 6N-HC1, and hydrogen sulfide was passed through for 1 hour. After filtering the deposited copper sulfide, the filtrate was washed with 1N-HC1, and then the hydrogen sulfide was removed by passing air through the resulting filtrate. The thus obtained solution was adjusted to pH 6.5 by conc. aqueous ammonia under ice-cooling, and the deposited crystalline product was filtered, washed with water and ethanol successively, and dried to afford 11.5 g (68 % of the theoretical value) of the desired product.

(Effect of the Invention)

The active esters of this invention are useful active esters with respect to acylating reagents and synthesis of peptides, as apparent from Working Examples and Reference Examples, and are proved to have excellent properties which are not possessed by the conventional active esters.

Also, in separation and removal aftger reaction, the active esters of this invention can be easily separated and removed from the desired product by washing with water, and afford a reaction yield equal or more, compared with the conventional active esters.

## Claims

(1) An active esterifying agent which is a reagent for synthesizing an active ester comprising using a sulfonium salt represented by the general formula:

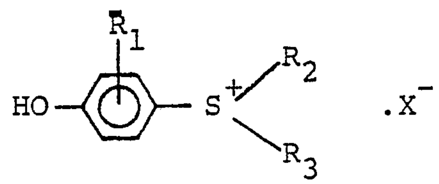

wherein $R_1$ represents a hydrogen, an alkyl group, or a halogen group; $R_2$ and $R_3$, which may be same or different, each represents an alkyl groupl and $X^-$ represents an anion typified by a halogen ion, a perchloric acid ion, a hydrogensulfuric acid ion, a methylsulfuric acid ion, and a p-toluenesulfonic acid ion.

(2) An active esterifying agent according to claim (1), wherein said sulfonium salt is 4-hydroxyphenyldimethylsulfonium perchlorate.

(3) An active esterifying agent according to claim (1), wherein said sulfonium salt is 4-hydroxyphenyldimethylsulfonium p-toluenesulfonate.

(4) An active esterifying agent according to claim (1), wherein said sulfonium salt is 4-hydroxyphenyldimethylsulfonium methylsulfate.

(5) An active esterifying agent according to claim (1), wherein said sulfonium salt is 4-hydroxyphenyldimethylsulfonium hydrogensulfate.

(6) A sulfonium compound represented by the general formula:

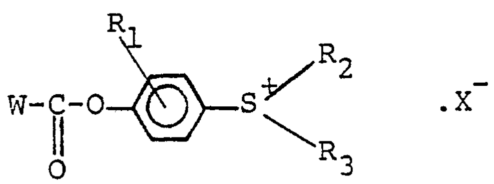

wherein $R_1$ represents a hydrogen, an alkyl group, or a halogen group; $R_2$ and $R_3$ which may be same or different, each represents an alkyl group; $X^-$ represents an anion typified by a halogen ion, a perchloric acid ion, a hydrogensulfuric acid ion, a methylsulfuric acid ion, and a p-toluenesulfonic acid ion; and W represents an alkyl group, alkoxy group,

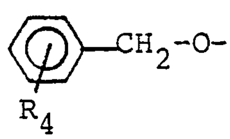 group

(in which $R_4$ represents a halogen, a hydrogen or a methoxy), or a $C_1$ - $C_5$ alkyl group substituted with 1 - 3 halogen atoms, an alkyl group or a fluorenylmethoxy group, or represents, together with $W\text{-}\underset{O}{\underset{\|}{C}}\text{-}$, an amino acid, a peptide, or a protein group, in which the terminal amino group and the side chain group of the amino acid has or has not a protective group.

(7) A sulfonium compound according to claim (6), wherein the sulfonium compound is 4-(tert-butyloxycarbonyloxy)phenyldimethysulfonium methylsulfate.

(8) A sulfonium compound according to claim (6), wherein the sulfonium compound is 4-(benzyloxycarbonyloxy)phenyldimethylsulfonium methylsulfate.

(9) A sulfonium compound according to claim (6), wherein the sulfonium compound is 4-(p-methoxybenzyloxy carbonyloxy)phenyldimethylsulfonium methylsulfate.

(10) A sulfonium compound according to claim (6), wherein the sulfonium compound is 4-(9-fluorenylmethyoxycarbonyloxy)phenyldimethylsulfonium methylsulfate.

(11) A sulfonium compound according to claim (6), wherein the sulfonium compound is 4-acetoxyphenyldimethylsulfonium methylsulfate.

(12) A sulfonium compound according to claim (6), wherein the sulfonium compound is 4-lauroylphenyldimethylsulfonium methylsulfate.

(13) An active ester for synthesizing an acid amide compound represented by the general formula:

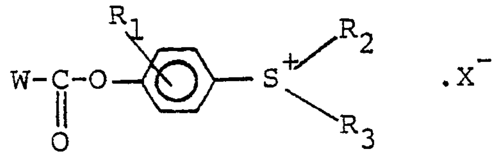

wherein $R_1$ represents a hydrogen, an alkyl group, or a halogen group; $R_2$ and $R_3$, which may be same or different, each represents an alkyl group; and $X^-$ represents an anion typified by a halogen ion, a perchloric acid ion, a hydrogen-sulfuric acid ion, a methylsulfuric acid ion, and a p-toluenesulfonic acid ion; and W represents an alkyl group, alkoxy group,

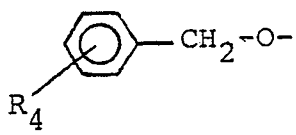

group .

(in which $R_4$ represents a halogen, a hydrogen or a methoxy), or a $C_1$ - $C_5$ alkyl group substituted with 1 - 3 halogen atoms, an alkyl group or a fluorenylmethoxy group, or represents, together with W- $\underset{\underset{\text{O}}{\|}}{\text{C}}$ -, an amino acid, a peptide, or a protein group, in which the terminal amino group and the side chain group of the amino acid has or has not a protective group.

(14) An active ester according to claim (13), wherein the active ester is 4-(tert-butyloxycarbonyloxy)-phenyldimethylsulfonium methylsulfate.

(15) An active ester according to claim (13), wherein the active ester is 4-(benzyloxycarbonyloxy)-phenyldimethylsulfonium methylsulfate.

(16) An active ester according to claim (13), wherein the active ester is 4-(p-methoxybenzyloxycarbonyloxy)phenyldimethylsulfonium methylsulfate.

(17) An active ester according to claim (13), wherein the active ester is 4-(9-fluorenylmethoxycarbonyloxy)phenyldimethylsulfonium methylsulfate.

(18) An active ester according to claim (13), wherein the active ester is 4-acetoxyphenyldimethylsulfonium methylsulfate.

(19) An active ester according to claim (13), wherein the active ester is 4-lauroylphenyldimethylsulfonium methylsulfate.

(20) An acylating agent represented by the general formula:

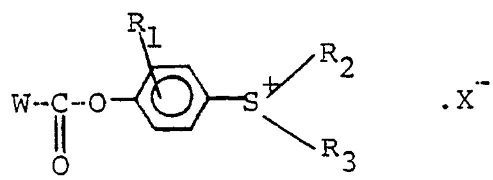

wherein $R_1$ represents a hydrogen, an alkyl group, or a halogen group; $R_2$ and $R_3$, which may be same or different, each represents an alkyl group; and $X^-$ represents an anion typified by a halogen ion, a perchloric acid ion, a hydrogen-sulfuric acid ion, a methylsulfuric acid ion and a p-toluenesulfonic acid ion; and W represents an alkyl group, alkoxy group,

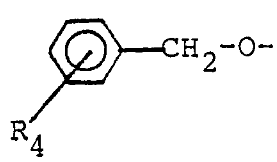

group

(in which $R_4$ represents a halogen, a hydrogen or a methoxy), or a $C_1$ - $C_5$ alkyl group substituted with 1 - 3 halogen atoms, an alkyl group or a fluorenylmethoxy group, or represents, together with $W\text{-}\underset{\underset{O}{\|}}{C}\text{-}$, an amino acid, a peptide, or a protein group, in which the terminal amino group and the side chain group of the amino acid has or has not a protective group.

(21) An acylating agent according to claim (20), wherein the acylating agent is 4-(tert-butyloxycarbonyloxy)phenyldimethylsulfonium methylsulfate.

(22) An acylating agent according to claim (20), wherein the acylating agent is 4-(benzyloxycarbonyloxy)phenyldimethylsulfonium methylsulfate.

(23) An acylating agent according to claim (20), wherein the acylating agent is 4-(p-methoxybenzyloxycarbonyloxy)phenyldimethylsulfonium methylsulfate.

(24) An acylating agent according to claim (20), wherein the acylating agent is 4-(9-fluorenylmethoxycarbonyloxy)phenyldimethylsulfonium methylsulfate.

(25) An acylating agent according to claim (20), wherein the acylating agent is 4-acetoxyphenyldimethylsulfonium methylsulfate.

(26) An acylating agent according to claim (20), wherein the acylating agent is 4-lauroylphenyldimethylsulfonium methylsulfate.

(27) A process for producing an amide represented by the general formula:

$W\text{-}\underset{\underset{O}{\|}}{C}\text{-}B\text{-}Y$ 3

by reacting an active ester represented by the general formula:

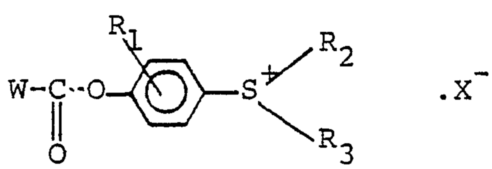

I

with a nuclephilic agent represented by the general formula:

H-B-Y 2

wherein $R_1$ represents a hydrogen, an alkyl group or a halogen group; $R_2$ and $R_3$, which may be same or different, each represents an alkyl group; $X^-$ represents an anion typified by a halogen ion, a perchloric acid ion, a hydrogen sulfuric acid ion, a methylsulfuric acid ion, and a p-toluenesulfonic acid ion; and W represents an alkyl group, alkoxy group,

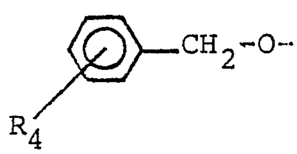

group,

(in which $R_4$ represents a halogen, a hydrogen or a methoxy), or a $C_1$ - $C_5$ alkyl group substituted with 1 - 3 halogen atoms, an aryl group or a fluorenylmethoxy group, or represents, together with $W\text{-}\underset{\underset{O}{\|}}{C}\text{-}$, an amino acid, a peptide, or a protein group, in which the terminal amino group and the side chain group of the amino acid has or has not a protective group, B represents a substituted or not-substituted imino group, Y represents an organic group or B and Y joint together to represent a heterocyclic amine. Alternatively H-B-Y represents an amino acid in which a proper protective group is or is not introduced into α-carboxyl group and/or side chain, or a peptide or protein in which a proper protective group is or is not introduced into the terminal carboxyl group and/or side chain.

(28) A process for producing an amide according to claim (27), in which the reaction of the active ester represented by the above general formula 1 with the nucleophilic agent represented by the above general formula 2 is conducted in water alone, or in an organic solvent having a compatibility with water alone, or in a mixed system of the above two.

(29) A process for producing an amide according to claim (27), in which the reaction of the active ester represented by the above general formula 1 with the nucleophilic agent represented by the above general formula 2 is conducted in an organic solvent having no compatibility with water alone or in two phase system of a solvent having no compatibility with water and water.

(30) A process for producing a monoamide represented by the general formula as defined in claim (27):

$W-\underset{\underset{O}{\parallel}}{C}-NH-Y'-NH_2$ 5

wherein Y′ represents a bivalent organic group, by reacting an active ester represented by the general formula 1 described above with a nucleophilic agent represented by the general formula:

$NH_2-Y'-NH_2$ 4

wherein Y′ has the same meanings as defined above, in an aqueous solvent while controlling the pH value in the solution.

(31) A process for producing δ-acylornithine as defined in claim (30) wherein the nucleophilic agent represented by the general formula 4 is ornithine and the reaction is conducted in an aqueous solution at pH of 5 or higher.

(32) A process for producing ε-acyllysine as defined in claim (30) wherein the nucleophilic agent represented by the general formula 4 is lysine and the reaction is conducted in an aqueous solution at pH of 5 or higher.

(33) A process for producing a peptide as defined in claim (27), wherein an active ester of an amino acid represented by the general formula 1 is reacted with an amino acid represented by the general formula 2 as described above.

(34) A process for producing a protected amino acid as defined in claim (27), wherein the active ester for the protective group of the amino acid represented by the general formula 1 is reacted with an amino acid represented by the general formula 2 described above.

(35) A process for producing a protected amino acid as defined in claim (34), wherein the protective group for the amino acid is benzyloxycarbonyl group, tert-butyloxycarbonyl group 9-fluorenylmethoxycarbonyl group, or p-methoxybenzyloxycarbonyl group.

(36) A process for producing an ε-acyllysine or δ-acylornithine with comprises reacting a sulfonium compound represented by the following general formula and a lysine or ornithine in an aqueous solution at a pH of 5 or higher:

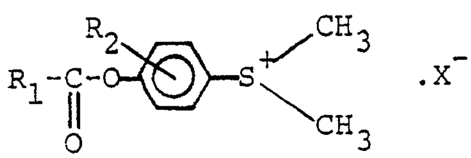

where $R_1$ represents alkyl group, halogenated alkyl group, tert-butyloxy group, benzyloxy group, p-methoxy-benzyloxy group, p-chlorobenzyloxy group, p-bromobenzyloxy group, $R_2$ represents hydrogen, alkyl group or halogen atom, $X^-$ represents anion typically represented by halogen ion, p-toluenesulfate ion, methylsulfate ion or hydrogensulfate ion.

(37) A production process as defined in Claim 36, wherein the sulfonium compound is used in an amount from 1 to 2 molar amount based on lysine or ornithine.

(38) A sulfonium compound represented by the general formula:

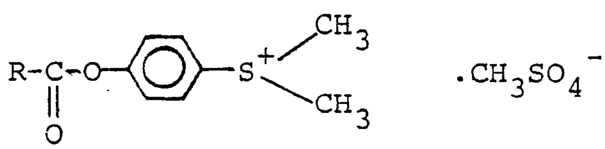

where R represents a long-chained alkyl group.

(39) An acylating agent represented by the general formula:

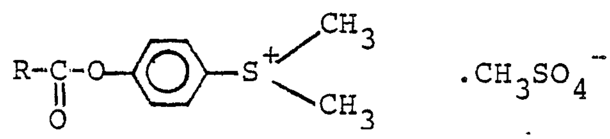

where R represents a long-chained alkyl group.

(40) A sulfonium compound represented by the general formula:

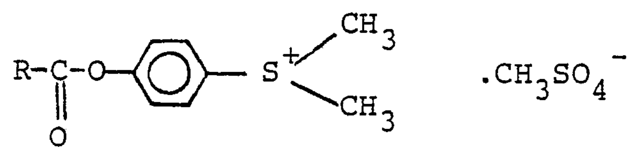

where R represents 9-fluorenylmethoxy group.

(41) A 9-fluorenylmethoxy carbonylating agent of a sulfonium compound represented by the following formula:

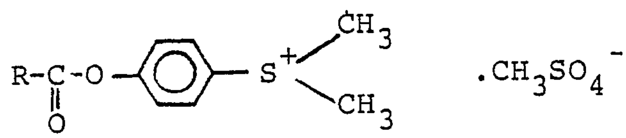

where R represents a 9-fluorenylmethoxy group.

European Patent Office

# EUROPEAN SEARCH REPORT

Application Number

EP 89 10 3237

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 814 453 (THE BOOTS CO)<br>* page 16, formula III *<br>--- | 1 | C 07 C 149/46<br>C 07 C 102/06<br>C 07 K 1/08 |
| P,X | CHEMICAL ABSTRACTS vol. 109, no. 11, 12th September 1988, page 770, abstract no. 93563t, Columbus, Ohio, US; I. AZUSE et al.: "One step synthesis of epsilon-N-benzyloxycarbonyl-L-lysine using water-soluble acylating reagent"; & CHEM. EXPRESS 1988, 3(1), 21-4<br>--- | 6,13,27,32 | |
| P,X | CHEMICAL ABSTRACTS vol. 109, no. 9, 29th August 1988, pages 736,737, abstract no. 73861k, Columbus, Ohio, US; K. KOUGE et al.: "Water-soluble active ester method. IV. A new benzyloxycarbonyl donor"; CHEM. EXPRESS 1987, 2(10), 583-6<br>--- | 1,6,13,27 | |
| X | CHEMICAL ABSTRACTS vol. 108, 6th - 13th June 1988, page 716, abstract no. 205060q, Columbus, Ohio, US; K. KOUGE et al.: "Peptide synthesis in aqueous solution. I. Application of p-(dialkylsulfonio)phenols as water-soluble coupling reagents"; & BULL. CHEM. SOC. JPN. 1987, 60(7), 2409-18<br>--- -/- | 1,27,30 | TECHNICAL FIELDS SEARCHED (Int. Cl.4)<br>C 07 C 149/46<br>C 07 C 102/06<br>C 07 K 1/08 |

The present search report has been drawn up for all claims

| Place of search | Date of completion of the search | Examiner |
|---|---|---|
| BERLIN | 26-05-1989 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 10 3237

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS vol. 108, no. 3, 18th January 1988, page 639, abstract no. 22245j, Columbus, Ohio, US; K. KOUGE et al.: "Water-soluble active ester method. III. Synthesis of FMRFamide, a molluscan neuropeptide"; & CHEM. EXPRESS 1987, 2(3), 153-6<br>--- | 27 | |
| X | CHEMICAL ABSTRACTS vol. 108, no. 3, 18th January 1988, page 639, abstract no. 22249p, Columbus, Ohio, US; K. KOUGE et al.: "Reactivity and application of p-dialkylsulfoniophenyl esters as a water-soluble active ester method for peptide synthesis"; & PEPT. CHEM. 1986 (Pub. 1987). 24th, 237-40<br>--- | 1,27 | |
| X | CHEMICAL ABSTRACTS vol. 107, no. 5, 3rd August 1987, page 755, abstract no. 40305n, Columbus, Ohio, US; K. KOUGE et al.: "Water-soluble active ester method. II. Synthesis of oligopeptides by using p-dialkylsulfoniophenyl esters"; & CHEM. EXPRESS 1986, 1(8), 499-502<br>--- | 1,6,13, 27 | TECHNICAL FIELDS SEARCHED (Int. Cl.4) |
| X | CHEMICAL ABSTRACTS vol. 106, no. 15, 13th April 1987, page 596, abstract no. 119381x, Columbus, Ohio, US; K. KOUGE et al.:"Water-soluble active ester method. I. Reactivity of p-dialkylsulfoniophenyl esters for acylation"; & CHEM. EXPRESS 1986, 1(7), 427-30<br>--- -/- | 1,6,13, 27 | |

The present search report has been drawn up for all claims

| Place of search | Date of completion of the search | Examiner |
|---|---|---|
| BERLIN | 26-05-1989 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 89 10 3237

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS vol. 104, 6th - 20th January 1986, page 511, abstract no. 5628m, Columbus, Ohio, US; & JP - A - 60 32762, 19-02-1985 | 6 | |
| A | CHEMICAL ABSTRACTS vol. 97, 20th - 27th December 1982, page 320, abstract no. 210356z, Columbus, Ohio, US; H.G. HEWITT et al.: "The effect of BTS 44584, a ternary sulfonium growth retardant, on net photosynthesis and yield in soybeans"; EASTER SCH. AGRIC. SCI., UNIV. NOTTINGHAM, (Proc.) 1981 (Pub. 1982). 33rd (Chem. Manipulation Crop Growth Dev.), 221-35 | 13,27 | |

TECHNICAL FIELDS SEARCHED (Int. Cl.4)

The present search report has been drawn up for all claims

| Place of search | Date of completion of the search | Examiner |
|---|---|---|
| BERLIN | 26-05-1989 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)